# EUROPEAN PATENT APPLICATION

(11) **EP 2 980 218 A1**
(43) Date of publication of application: **03.02.2016**
(21) Application number: 14179135.0
(22) Date of filing: 30.07.2014
(51) Int. Cl.: C12N 15/115, C07K 14/745, A61K 31/7088

(54) **Aptamer thrombin complex for use as an antidote to direct acting thrombin inhibitors**

(71) Applicant: Rheinische Friedrich-Wilhelms-Universität Bonn, 53113 Bonn (DE)
(72) Inventor: Pötzsch, Bernd, 53129 Bonn (DE); Müller, Jens, 53119 Bonn (DE); Mayer, Günter, 53123 Bonn (DE)
(74) Representative: Michalski Hüttermann & Partner Patentanwälte mbB

(57) **Abstract**

The present invention relates to a complex formed by thrombin and a thrombin-binding aptamer, said complex is capable of neutralizing the activity of a direct acting thrombin inhibitor, and to a use as an antidote to blood anticoagulants selected from direct acting thrombin inhibitors.

SEQUENCE LISTING

<110>  Rheinische Friedrich-Wilhelms Universität Bonn

<120>  Aptamer thrombin complex for use as an antidote to direct acting
       thrombin inhibitors

<130>  UD 40503/SAM

<160>  5

<170>  PatentIn version 3.3

<210>  1
<211>  59
<212>  DNA
<213>  Artificial

<220>
<223>  aptamer



<220>
<221>  misc_feature
<222>  (16)..(31)
<223>  optionally at least one of the adenosine nucleotides is replaced
       by a thymidine nucleotide

<400>  1
ggttggtgtg gttggaaaaa aaaaaaaaaa agtccgtggt agggcaggtt ggggtgact      59


<210>  2
<211>  27
<212>  DNA
<213>  Artificial

<220>
<223>  aptamer



<220>
<221>  misc_feature
<222>  (21)..(27)
<223>  N represents t or a deletion

<400>  2
ggttggtgtg gttggttttt nnnnnnn                                          27


<210>  3
<211>  59
<212>  DNA
<213>  Artificial

<220>
<223>  aptamer

<400>  3
ggttggtgtg gttggaaaaa aataaaaaaa agtccgtggt agggcaggtt ggggtgact      59


<210>  4

<211>  25
<212>  DNA
<213>  Artificial

<220>
<223>  aptamer

<400>  4
ggttggtgtg gttggttttt ttttt                                          25


<210>  5
<211>  15
<212>  DNA
<213>  Artificial

<220>
<223>  aptamer

<400>  5
ggttggtgtg gttgg                                                     15

## Description

The present invention relates to the field of anticoagulant therapy.

An increasing number of patients in the Western countries require long-term and continuous anticoagulant treatment for the prevention and treatment of thromboembolic disorders such as deep vein thrombosis, lung embolism, and cardioembolic stroke. Vitamin K-antagonists are widely and successfully employed for this indication, although a most important disadvantage is the incidence of major bleeding complications including fatal intracranial haemorrhage. To overcome these shortcomings several novel anticoagulant drugs including dabigatran and dabigatran etexilate have been developed. The direct acting thrombin inhibitor (DTI) dabigatran is a new generation anticoagulant approved for the prevention and treatment of thromboembolic diseases in the US and Europe. Clinical studies demonstrate an improved efficacy-to-safety index compared to vitamin K-antagonists, but as with all other anticoagulants, bleeding remains a major problem.

Thrombin is a favourable target in the development of new anticoagulants as it is the key enzyme of hemostasis. Hence, besides dabigatran several DTIs for parenteral use have been developed and are in clinical use so far including the hirudin derivative bivalirudin, and the arginine derivate argatroban. Additional oral DTIs are in development or tested in clinical studies. These thrombin inhibiting anticoagulants are successfully used in the prevention and treatment of thrombotic diseases. The use of DTIs, however, can be complicated by the occurrence of major and even life-threatening hemorrhagic complications requiring urgent reversal of the anticoagulant effect of the DTIs. This is of particular importance for the oral DTI dabigatran, because of its relative long half-life time and its elimination through the renal route. Although efforts were made to develop neutralising antibodies usable as a specific antidote they are not available until now. US 2012/0027780 A1 for example discloses an antibody molecule capable of neutralizing the activity of an anticoagulant. Moreover, nothing is known about potential side effects through formation of dabigatran-antibody complexes and possible interactions of these antibodies with other cellular and molecular elements of the human body, possibly inducing a generalized inflammatory response or other side effects. Moreover, each of these antibodies will be specific for only one of the DTIs.

In search for a reversal strategy, the efficacy of unspecific hemostatic agents including prothrombin complex concentrates, activated factor VIIa, and activated prothrombin complex concentrates have been tested in various animal models and in volunteer studies. The results however are conflicting and there is no clear evidence that one of these agents can be successfully used in emergency situations. Major bleeding complications still are a potential side effect of dabigatran treatment and other DTIs. In the absence of a specific antidote strategy these bleeding complications are difficult to manage. Therefore, there is a need to provide antidotes for anticoagulant therapy.

Therefore, the object underlying the present invention was to provide an antidote to direct acting thrombin inhibitors.

The problem is solved by a complex formed by thrombin and a thrombin-binding aptamer comprising a nucleotide sequence selected from the group comprising 5'-GGTTGGTGTGGTTGGAAAAAAAAGTCCGTGGTA-GGGCAGGTTGGGGTGACT-3' (SEQ ID NO: 1) wherein optionally at least one of the adenosine nucleotides at positions 16 to 31 is replaced by a thymidine nucleotide and 5'-GGTTGGTGTGGTTGGTTTTTNNNNNNN-3' (SEQ ID NO: 2) wherein N represents a thymidine nucleotide or a deletion or pharmaceutically acceptable salts thereof capable of neutralizing the activity of a direct acting thrombin inhibitor.

Furthermore, the invention relates to a complex formed by thrombin and a thrombin-binding aptamer comprising a nucleotide sequence selected from the group comprising 5'-GGTTGGTGTGGTTGGAAAAAAAAGTCCGTGGTAGGGCAGGTTG-GGGTGACT-3' (SEQ ID NO: 1) wherein optionally at least one of the adenosine nucleotides at positions 16 to 31 is replaced by a thymidine nucleotide, 5'-GGTTGGTGTGGTTGGTTTTTNNNNNNN-3' (SEQ ID NO: 2) wherein N represents a thymidine nucleotide or a deletion and 5'-GGTTGGTGTGGTTGG-3' (SEQ ID NO: 5) or pharmaceutically acceptable salts thereof, for use as an antidote to blood anticoagulants selected from direct acting thrombin inhibitors.

Surprisingly it was found that the aptamer-complexed thrombin looses its biological functions but retains full reactivity towards direct acting thrombin inhibitors. Advantageously, the aptamer effectively inhibits the biological functions of thrombin by binding with high binding affinity and specificity to the thrombin exosite I or to exosites I and II but does not block the active centre of thrombin, thus allowing interaction of thrombin bound to the aptamer with thrombin inhibitors. The aptamer-complexed thrombin completely abolishes the prothrombotic and procoagulant activities of thrombin, but does not negatively affect the reactivity towards direct acting thrombin inhibitors. Beneficially, the aptamer thrombin complex provides a safe antidote for direct acting thrombin inhibitors and avoids unwanted side effects of a thrombin infusion ranging from the induction of a bleeding phenotype at low nanomolar doses to the occurrence of life-threatening thromboembolic complications and generalized microvascular thrombosis at higher dosages.

As used herein, the term "aptamer" refers to a single-stranded oligonucleotide that recognises its target with high specificity and binds to the target with high affinity in the low nanomolar range. The aptamer can be provided in the form of a single-stranded DNA or RNA molecule. As will be obvious to a person of ordinary skills in the art, if the nucleic acid is an RNA molecule the thymidine or "T" in the nucleotide sequence is to be read as meaning "U" or uridine. Preferably, the aptamer comprises a deoxyribonucleotide sequence. DNA aptamers can exhibit better stability. The nucleotides of a DNA molecule may comprise a chemical modification such as a locked nucleic acid (LNA) or a substituent. Preferred substituents are selected from the group comprising fluorine, C₁-C₅-alkoxy particularly methoxy, or an amino group.

As used herein, the term "direct acting thrombin inhibitor" (DTI) refers to a class of anticoagulants that act by directly inhibiting the enzyme thrombin.

As used herein, the term "thrombin" refers to the active enzyme thrombin that is generated by proteolytic cleavage of the zymogen prothrombin. The enzyme complex prothrombinase catalyzes the proteolysis of two peptide bonds in prothrombin, which gives rise to an NH₂-terminal derived F1.2 region and the heterodimer alpha-thrombin. In alpha-thrombin an "A" chain (6000 Da) is covalently linked to a "B" chain (31,000 Da) through a single disulfide bond.

Recombinant human thrombin is commercially available. Also human alpha-thrombin prepared from human plasma is commercially available. As the direct acting thrombin inhibitor will bind to any mammalian thrombin, also other mammalian thrombins are usable, for example bovine, murine, rabbit or rat thrombin. To minimize side-effects, human thrombin is preferred. Thrombin can be provided by approved drugs such as prothrombin complex concentrate (PCC) or activated PCC (FEIBA NF), an anti-inhibitor coagulant complex, available at Baxter Corporation.

The aptamer thrombin complex is capable of neutralizing the activity of direct acting thrombin inhibitors. In other words, the aptamer thrombin complex is able to reverse the effects of the anticoagulant effect of direct acting thrombin inhibitors in plasma. The aptamer thrombin complex provides an antidote allowing efficient and safe reversal of the anticoagulant effect of direct acting thrombin inhibitors that can be universally used for all direct acting thrombin inhibitors currently under clinical use. In preferred embodiments, the direct acting thrombin inhibitor is selected from the group comprising dabigatran, dabigatran etexilate and/or argatroban.

A preferred direct acting thrombin inhibitor in the context of the present invention is dabigatran (CAS 211914-51-1), also denoted N-[2-(4-Aminoiminomethylphenylamino)-1-methyl-1H-benzimidazol-5-ylcarbonyl]-N-(2-pyridyl)-beta-alanine (C₂₅H₂₅N₇O₃, Mᵣ = 471.5 g/mol). Dabigatran usually is applied as a prodrug denoted dabigatran etexilate (CAS 211915-06-9) that is according to IUPAC nomenclature also denoted ethyl 3-[[[2-[[[4-[[[(hexyloxy)carbonyl]amino]iminomethyl]phenyl]amino]methyl]-1-methyl-1H-benzimidazol-5-yl]carbonyl](pyridin-2-yl)amino]propanoate. Dabigatran etexilate is converted into the active compound dabigatran after entering the body. Dabigatran etexilate is an oral available direct acting thrombin inhibitor (DTI) approved in Europe and in the US for a variety of indications including prophylaxis and treatment of deep vein thrombosis, and prevention of cardioembolic complications. Dabigatran etexilate is available under the trade name Pradaxa® at Boehringer Ingelheim. A preferred polymorph of dabigatran etexilate is dabigatran etexilate mesylate.

A further preferred direct acting thrombin inhibitor is argatroban. Argatroban is denoted (2*R*,4*R*)-1-[(2*S*)-5-(diaminomethylideneamino)-2-[[(3*R*)-3-methyl-1,2,3,4-tetrahydroquinolin-8-yl]sulfonylamino]pentanoyl]-4-methyl-piperidine-2-carboxylic acid according to IUPAC nomenclature and is obtainable under the trade name Argatra® from Mitsubishi Pharma. The capability of neutralizing the activity of direct acting thrombin inhibitors, at least experimentally, also may be of advantage in reversing the effects of direct acting thrombin inhibitors that currently are not under clinical use such as melagatran, ximelagatran, hirudin, bivalirudin, lepirudin or desirudin. Ximelagatran, ethyl 2-[[(1*R*)-1-cyclohexyl-2-[(2*S*)-2-[[4-[(Z)-N'-hydroxycarbamimidoyl]phenyl]methylcarbamoyl]azetidin-1-yl]-2-oxoethyl]amino]acetate, is a direct thrombin inhibitor that is converted in vivo to the active agent melagatran. Hirudin is a polypeptide direct thrombin inhibitor with a very high affinity for human thrombin. Bivalirudin, lepirudin and desirudin are recombinant hirudin-based anticoagulants.

A stable aptamer thrombin complex is formed between thrombin and the aptamers via van der Waals forces, hydrogen bonding and/or electrostatic interaction. A complex readily forms upon incubation of thrombin with the aptamer. However, it is preferred to enforce the binding of the aptamers to thrombin via a covalent linkage. In a preferred embodiment, the aptamer of SEQ ID NO: 1 or SEQ ID NO: 2 and thrombin are additionally covalently linked. An additional covalent linkage can be provided by introduction of a carboxyl-group (COOH) into the aptamer sequence. This modification can be easily done during aptamer synthesis by incorporation of a carboxy-dT-residue into the sequence. Modified aptamers then can be covalently bound to an amino-group present on the surface of the thrombin molecule via an amine-reactive intermediate N-hydroxy-succinimide (NHS) ester. During a first step, the carboxyl-group of the aptamer can be reacted to an amine-reactive NHS ester in the presence of a carbodiimide such as 1-ethyl-3-[3-dimethylaminopropyl]carbodiimide (EDC). After incubation, free reaction components may be removed from the activated aptamers and thrombin can be added to allow for the covalent binding.

It is preferred that the modification for providing a covalent linkage is present in a part of the aptamer that is not binding to exosites I and II of thrombin. It is hence preferred that at least one of the adenosine nucleotides at positions 16 to 31, also denoted poly-dA-linker region, of the aptamer of SEQ ID NO: 1 is replaced by a thymidine nucleotide, particularly a carboxylated thymidine nucleotide. Preferably, the aptamer of SEQ ID NO: 1 is modified with a single carboxy-dT within the poly-dA-linker region, replacing one of the dA nucleotides. A preferred carboxylated sequence is the carboxylated SEQ ID NO: 3 e.g. 5'-GGTTGGTGTGGTTGGAAAAAAA(carboxy-dT)AAAAAAA-AGTCCGTGGTAGGGCAGGTTGGGGTGACT-3'. It is preferred that one of the adenosine nucleotides in the centre of the poly-dA-linker region is replaced by a thymidine, for example an adenosine at position 20 to 26.

It is further preferred that the aptamer of SEQ ID NO: 2, 5'-GGTTGGTGTGGTTGGTTTTTNNNNNNN-3' (SEQ ID NO: 2), comprises 5 to 12, preferably 6 to 10, most preferred 10 thymidine nucleotides at the 3'-end. The additional thymidine nucleotides are also denoted dT-linker sequence. Preferably, the aptamer of SEQ ID NO: 2 is modified with a single carboxy-dT at the 3'-end of an additional dT-linker sequence that is added to the 3'-end of the original aptamer sequence. A preferred carboxylated sequence is carboxylated SEQ ID NO: 4 given as follows: 5'- GGTTGGTGTGGTTGGTTTTTTTTT-carboxy-dT-3'. Advantageously, the aptamers of SEQ ID NO: 3 and SEQ ID NO: 4 can still bind with high binding affinity to thrombin.

In preferred embodiments, the aptamer hence has a nucleotide sequence selected from the group comprising 5'-GGTTGGTGTGGTTGGAAAAAAATAAAAAAAAGTCCGTGGTA-GGGCAGGTTGGGGTGACT-3' (SEQ ID NO: 3) and 5'-GGTTGGTGTGGTTGGTTTTTTTTTT-3' (SEQ ID NO: 4). The complex formed by thrombin and the aptamer of SEQ ID NO: 3 exhibits a particularly high binding affinity of *K*_{d} = 0.65 nM.

A further aspect of the present invention relates to a complex formed by thrombin and a thrombin-binding aptamer comprising a nucleotide sequence selected from the group comprising 5'-GGTTGGTGTGGTTGGAAAAAAAAAAAAAAAAGTCCGTGGTA-GGGCAGGTTGGGGTGACT-3' (SEQ ID NO: 1) wherein optionally at least one of the adenosine nucleotides at positions 16 to 31 is replaced by a thymidine nucleotide and 5'-GGTTGGTGTGGTTGGTTTTTNNNNNNN-3' (SEQ ID NO: 2) wherein N represents a thymidine nucleotide or a deletion or pharmaceutically acceptable salts thereof, for use as a medicament.

Advantageously, the aptamer-complexed thrombin looses its biological functions but retains full reactivity towards direct acting thrombin inhibitors. The aptamer thrombin complex provides a safe antidote that is universally usable for direct acting thrombin inhibitors currently under clinical use.

A further aspect of the present invention relates to a complex formed by thrombin and a thrombin-binding aptamer comprising a nucleotide sequence selected from the group comprising 5'-GGTTGGTGTGGTTGGAAAAAAAAAAAAAAAA-GTCCGTGGTAGGGCAGGTTGGGGTGACT-3' (SEQ ID NO: 1) wherein optionally at least one of the adenosine nucleotides at positions 16 to 31 is replaced by a thymidine nucleotide, 5'-GGTTGGTGTGGTTGGTTTTTNNNNNNN-3' (SEQ ID NO: 2) wherein N represents a thymidine nucleotide or a deletion and 5'-GGTTGGTGTGGTTGG-3' (SEQ ID NO: 5) or pharmaceutically acceptable salts thereof, for use as an antidote to blood anticoagulants selected from direct acting thrombin inhibitors.

The ability of the aptamer thrombin complex to titrate the direct acting thrombin inhibitors dabigatran and argatroban presently in clinical use was demonstrated in in-vitro plasma-based studies. Moreover, the ability of the aptamer thrombin complex to antagonize the anticoagulant functions was demonstrated in vivo in the mouse model. Advantageously, a prothrombotic response was not induced. Further, it was found that no side-effects through activation of the haemostatic system by remaining procoagulant active thrombin were induced. This shows that the aptamer thrombin complex is usable as a safe antidote to blood anticoagulants selected from direct acting thrombin inhibitors.

Particularly, the aptamer thrombin complex is usable in the therapeutic and/or prophylactic treatment of a bleeding or overdosing event in anticoagulant therapy.

As used herein, the term "prophylactic treatment" refers to either preventing or inhibiting the development of a clinical condition or disorder or delaying the onset of a pre-clinically evident stage of a clinical condition or disorder such as a bleeding. The term "prophylactic treatment" according to the invention is to be understood as meaning that the complex according to the invention can be applied before a bleeding is manifest. Especially, the term "prophylactic treatment" is to be understood as meaning a medical treatment.

Advantageously, the aptamer thrombin complex is usable as a specific reversal agent to prevent bleeding complications including life-threatening haemorrhage. The aptamer thrombin complex hence enables urgent surgical interventions that otherwise are difficult to manage with patients receiving anticoagulant therapy for the prevention and treatment of thromboembolic diseases. Also, the aptamer thrombin complex can counteract bleeding complications in case of overdosing events in anticoagulant therapy with direct acting thrombin inhibitors. Preferably, the direct acting thrombin inhibitors are selected from the group comprising dabigatran, dabigatran etexilate and/or argatroban.

Preferably, the aptamer thrombin complex formed between thrombin and the aptamer of is based on van der Waals forces, hydrogen bonding and/or electrostatic interaction, and additionally on a covalent linkage. In a preferred embodiment, the aptamer of SEQ ID NO: 1 or SEQ ID NO: 2 and thrombin additionally are covalently linked. An additional covalent linkage can be provided by introduction of a carboxyl-group (COOH) into the aptamers and the application of EDC/NHS-chemistry for covalent binding. In preferred embodiments, the aptamer has a nucleotide sequence selected from the group comprising 5'-GGTTGGTGTGGTTGGAAAAAAATAAAAAAAAGTCCGTGGTAGGGCAGGTTG-GGGTGACT-3' (SEQ ID NO: 3) and 5'-GGTTGGTGTGGTTGGTTTTTTTTTT-3' (SEQ ID NO: 4). A preferred carboxylated sequence SEQ ID NO: 3 is given as follows: 5'-GGTTGGTGTGGTTGGAAAAAAA(carboxy-dT)AAAAAAA-AGTCCGTGGTAGGGCAGGTTGGGGTGACT-3'. A preferred carboxylated sequence SEQ ID NO: 4 is given as follows: 5'- GGTTGGTGTGGTTGGTTTTTTTTT-carboxy-dT-3'. Also a complex formed by thrombin and the unmodified aptamer of SEQ ID NO: 5, 5'-GGTTGGTGTGGTTGG-3', is usable as an antidote to direct acting thrombin inhibitors. In embodiments, a dosage ranging from 0.1 to 10 mg/kg body weight may be usable to neutralise 1 µM of the active anticoagulant such as dabigatran.

The aptamers also are usable in form of pharmaceutically acceptable salts thereof. The term "pharmaceutically acceptable salts" refers to salts prepared from pharmaceutically acceptable non-toxic bases or acids. A pharmaceutically acceptable salt can be conveniently prepared from pharmaceutically acceptable non-toxic bases, including inorganic bases and organic bases. Preferred salts derived from inorganic bases include ammonium, calcium, magnesium, potassium and sodium salts. Salts derived from pharmaceutically acceptable organic non-toxic bases include salts of primary, secondary, and tertiary amines, as well as cyclic amines. Preferably, the pharmaceutically acceptable salt is selected from the group of sodium or potassium salts. Also, calcium or magnesium salts can be preferred.

For use as a medicament the aptamer thrombin complex can be used or included in a composition. Particularly for use as a medicament the aptamer thrombin complex can be used or included in a pharmaceutical composition. Accordingly, in another aspect the present invention relates to a pharmaceutical composition comprising as an active ingredient a complex formed by thrombin and a thrombin-binding aptamer comprising a nucleotide sequence selected from the group comprising 5'-GGTTGGTGTGGTTGG-AAAAAAAAAAAAAAAAGTCCGTGGTAGGGCAGGTTGGGGTGACT-3' (SEQ ID NO: 1) wherein optionally at least one of the adenosine nucleotides at positions 16 to 31 is replaced by a thymidine nucleotide and 5'-GGTTGGTGTGGTTGGTTTTTNNNNNNN-3' (SEQ ID NO: 2) wherein N represents a thymidine nucleotide or a deletion or pharmaceutically acceptable salts thereof.

Particularly, the present invention relates to a pharmaceutical composition comprising as an active ingredient a complex formed by thrombin and a thrombin-binding aptamer comprising a nucleotide sequence selected from the group comprising 5'-GGTTGGTGTGGTTGG-AAAAAAAAAAAAAAAAGTCCGTGGTAGGGCAGGTTGGGGTGACT-3' (SEQ ID NO: 1) wherein optionally at least one of the adenosine nucleotides at positions 16 to 31 is replaced by a thymidine nucleotide, 5'-GGTTGGTGTGGTTGGTTTTTNNNNNNN-3' (SEQ ID NO: 2) wherein N represents a thymidine nucleotide or a deletion and 5'-GGTTGGTGTGGTTGG-3' (SEQ ID NO: 5) or pharmaceutically acceptable salts thereof, for use as an antidote to blood anticoagulants selected from direct acting thrombin inhibitors. The pharmaceutical composition particularly is usable in the therapeutic and/or prophylactic treatment of a bleeding or overdosing event in anticoagulant therapy.

The pharmaceutical composition may be produced under sterile conditions using standard pharmaceutical techniques well known to those skilled in the art. For compositions convenient pharmaceutical media may be employed. For example, water, glycols, oils, alcohols and the like may be used to form liquid preparations such as solutions. The composition may comprise a pharmaceutical carrier, which can be, for example, a solid, liquid, or gas. Suitable carriers preferably are liquid and correspond to the substances ordinarily employed in formulation technology for pharmaceutical formulations. The compositions can be suitable for oral, dermal, rectal, topical, and parenteral administration. The compositions particularly can be suitable for parenteral administration, including subcutaneous, intramuscular, and intravenous administration.

The pharmaceutical composition of the present invention can be presented as discrete unit suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion or as a water-in-oil liquid emulsion. Compositions suitable for parenteral administration may be prepared as a solution or suspension of the aptamer thrombin complex in water. Compositions suitable for injection include sterile aqueous solutions or dispersions.

In preferred embodiments, the aptamer has a nucleotide sequence selected from the group comprising 5'-GGTTGGTGTGGTTGGAAAAAAATAAAAAAAAGTCCGTGGTA-GGGCAGGTTGGGGTGACT-3' (SEQ ID NO: 3) and 5'-GGTTGGTGTGGTTGGTTTTTTTTTT-3' (SEQ ID NO: 4). A preferred carboxylated sequence SEQ ID NO: 3 is given as follows: 5'-GGTTGGTGTGGTTGGAAAAAAA(carboxy-dT)AAAAAAA-AGTCCGTGGTAGGGCAGGTTGGGGTGACT-3'. A preferred carboxylated sequence SEQ ID NO: 4 is given as follows: 5'- GGTTGGTGTGGTTGGTTTTTTTTT-carboxy-dT-3'. In preferred embodiments, the direct acting thrombin inhibitor is selected from the group comprising dabigatran, dabigatran etexilate and/or argatroban. In embodiments, a dosage ranging from 0.1 to 10 mg/kg body weight may be used to neutralise 1 µM of the active anticoagulant such as dabigatran.

A further aspect of the present invention also relates to the use of a complex formed by thrombin and a thrombin-binding aptamer comprising a nucleotide sequence selected from the group comprising 5'-GGTTGGTGTGGTTGGAAAAAAAAAAAAAAAA-GTCCGTGGTAGGGCAGGTTGGGGTGACT-3' (SEQ ID NO: 1) wherein optionally at least one of the adenosine nucleotides at positions 16 to 31 is replaced by a thymidine nucleotide and 5'-GGTTGGTGTGGTTGGTTTTTNNNNNNN-3' (SEQ ID NO: 2) wherein N represents a thymidine nucleotide or a deletion or a pharmaceutically acceptable salt thereof, for the manufacture of a medicament. The present invention particularly relates to the use of a complex formed by thrombin and a thrombin-binding aptamer comprising a nucleotide sequence selected from the group comprising 5'-GGTTGGTGTGGTTGG-AAAAAAAAAAAAAAAAGTCCGTGGTAGGGCAGGTTGGGGTGACT-3' (SEQ ID NO: 1) wherein optionally at least one of the adenosine nucleotides at positions 16 to 31 is replaced by a thymidine nucleotide, 5'-GGTTGGTGTGGTTGGTTTTTNNNNNNN-3' (SEQ ID NO: 2) wherein N represents a thymidine nucleotide or a deletion and 5'-GGTTGGTGTGGTTGG-3' (SEQ ID NO: 5) or pharmaceutically acceptable salts thereof, for the manufacture of a medicament for use as an antidote to blood anticoagulants selected from direct acting thrombin inhibitors. Particularly, the aptamer thrombin complex is usable for the therapeutic and/or prophylactic treatment of a bleeding or overdosing event in anticoagulant therapy.

A further aspect of the present invention relates to a method for preventing or treating a bleeding or overdosing event in anticoagulant therapy, the method comprising the step of administering to a subject a therapeutically effective amount of a complex formed by thrombin and a thrombin-binding aptamer comprising a nucleotide sequence selected from the group comprising 5'-GGTTGGTGTGGTTGGAAAAAAAAAAAAAAAA-GTCCGTGGTAGGGCAGGTTGGGGTGACT-3' (SEQ ID NO: 1) wherein optionally at least one of the adenosine nucleotides at positions 16 to 31 is replaced by a thymidine nucleotide, 5'-GGTTGGTGTGGTTGGTTTTTNNNNNNN-3' (SEQ ID NO: 2) wherein N represents a thymidine nucleotide or a deletion and 5'-GGTTGGTGTGGTTGG-3' (SEQ ID NO: 5) or pharmaceutically acceptable salts thereof. The subject may be a mammal, preferably the subject is a human. The term "therapeutically effective amount" is used herein to mean an amount or dose sufficient to cause an improvement in a clinically significant condition in the subject. In embodiments, the therapeutically effective amount may be a dosage ranging from 0.1 to 10 mg/kg body weight. Such amount of the aptamer thrombin complex is usable to neutralise 1 µM of the active anticoagulant such as dabigatran.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:

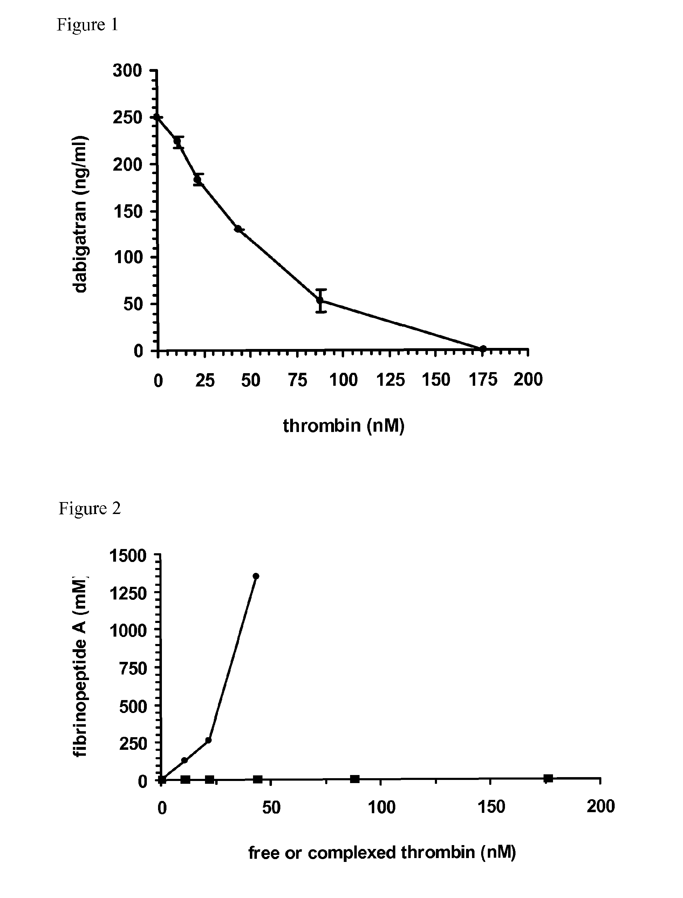

- Figure 1: The reversal of the anticoagulant activity of dabigatran using human α-thrombin. Dabigatran containing plasma was mixed with concentrations of 11 nM, 22 nM, 44 nM, 88 nM, and 176 nM of human α-thrombin.Data are given as mean ± standard deviation of three different experiments.
- Figure 2: The formation of fibrinopeptide A from fibrinogen in citrate anti-coagulated plasma samples upon addition of 11 nM, 22 nM and 44 nM of human α-thrombin (shown as dots) and 11 nM, 22 nM and 44 nM, 88 nM, and 176 nM of aptamer-thrombin complex (shown as squares).

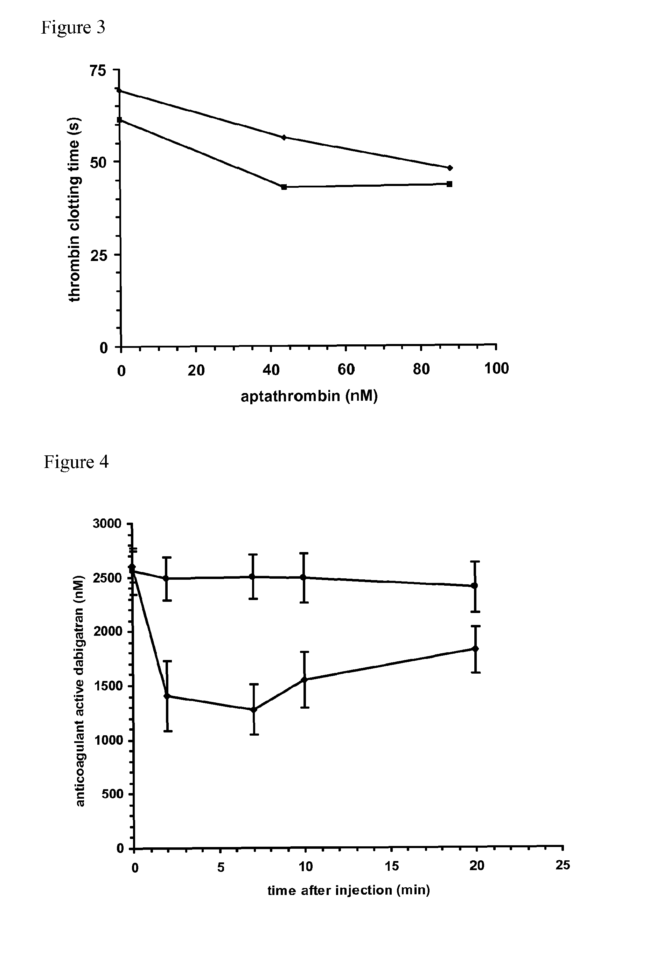

- Figure 3: The dose-dependent reversal of the anticoagulant activity of argatroban and dabigatran in plasma by addition of 44 nM and 88 nM of the aptamer thrombin complex (aptathrombin) to plasma containing the DTIs argatroban (squares) and dabigatran (rhombs) measured as a decrease of the thrombin clotting time.
- Figure 4: The efficacy and safety of the aptamer thrombin complex in the mouse model. Healthy mice were fed with dabigatran and treated with the aptamer thrombin complex (n=5) (rhombs) or buffer control (dots) (n=3) at t=0. The anticoagulant activity of dabigatran was determined for 20 min by taking blood samples and measuring the residual blood levels of functional active dabigatran. Data are given as mean ± standard deviation.

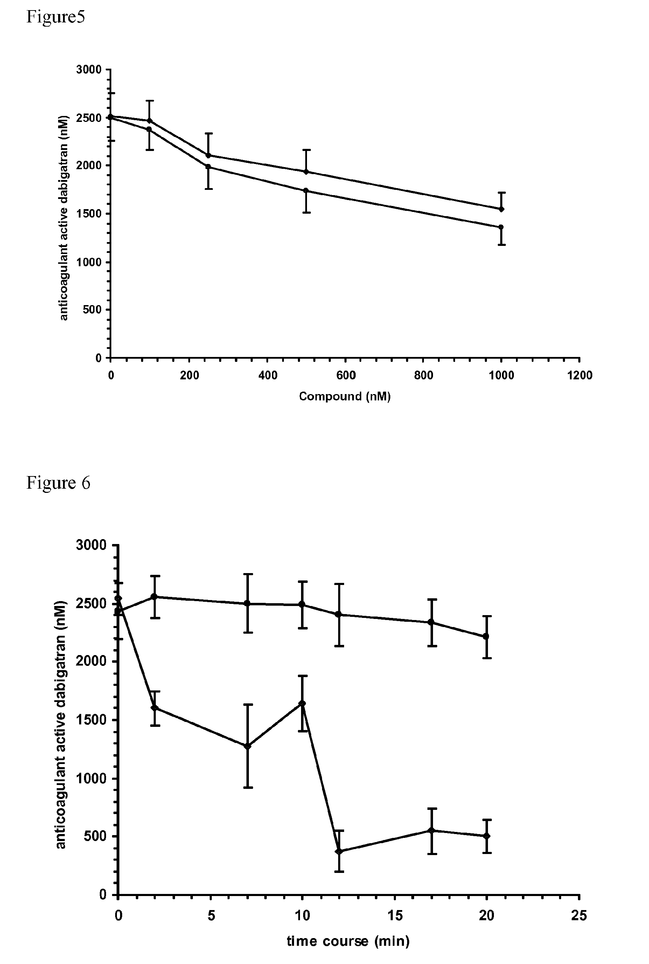

- Figure 5: The dose-effect relationship of aptathrombin in vivo. Dabigatran anticoagulated mice were treated with aptathrombin in increasing doses, and after 2 min (dots) und 7 min (rhombs) the anticoagulant activity of dabigatran in the plasma was determined. Data are given as mean ± standard deviation for two mice per determination.
- Figure 6: The effect of repeated administration of aptathrombin. Plotted is the level of anticoagulant active dabigatran of dabigatran anticoagulated mice (n=3) which received aptathrombin at t=0 and t=10 min (rhombs), and control mice (n=2) (dots) which received only buffer for a time period of 20 minutes. Data are given as mean ± standard deviation.

### Reagents and materials:

Human alpha-thrombin was obtained from CellSystems (St. Katharinen, Germany). Dabigatran (Pradaxa®) was obtained from Boehringer Ingelheim, Germany. Argatroban (Argatra®) was obtained from Mitsubishi Pharma (Düsseldorf, Germany). Native aptamers and carboxy-dT-modified aptamers were synthesized and purified by Microsynth (Balgach, Switzerland) and Eurogentec (Seraing, Belgium), respectively.

### Direct thrombin inhibitor assay:

Plasma levels of anticoagulant active direct thrombin inhibitors (DTIs) were measured using a direct thrombin inhibitor assay obtained from Hyphen, as described by Love et al., Thromb Haemost 98; 234-242, 2007. Direct thrombin inhibitors-containing plasma or the DTI containing reaction mixture was diluted 1:8 using Owens buffer or imidazole buffer. One volume of this prediluted plasma was added to two volumes of pooled normal plasma and incubated for 60 s at 37°C. After adding 100 µl of thrombin reagent the clotting time was recorded using a BCS-XP (Siemens Healthcare). Standard curves for dabigatran (0-0.5 µg/ml) and argatroban (0.1-6 µg/ml) were prepared using commercially available controls or drugs. Controls run before and after each standard curve showed similar values.

### Example 1

### Construction of a covalently linked aptamer-thrombin complex (aptathrombin)

100 µM of the carboxylated aptamer of nucleotide sequence SEQ ID NO:3, 5'-GGTTGGTGTGGTTGGAAAAAAA(carboxy-dT)AAAAAAAAGTCCGTGGTA-GGGCAGGTTGGGGTGACT-3', or 100 µM of the carboxylated aptamer of nucleotide sequence SEQ ID NO:4; 5'- GGTTGGTGTGGTTGGTTTTTTTTT-carboxy-dT-3', were incubated for 15 min at room temperature with in 0.1 M MES-buffer (0.5 M NaCl, pH 6.0) containing 2 mM 1-ethyl-3-[3-dimethylaminopropyl]carbodiimide (EDC) and 5 mM Sulfo-NHS (NHS: N-hydroxy-succinimide) in a total volume of 50 µl. Subsequently, the modified aptamer was purified by gelfiltration. For this purpose, a MicroSpin G-25 column (GE Healthcare, Solingen, Germany) was equilibrated with 50 µl PBS, pH 7.4, and the sample was separated by centrifugation at 750 g for 2 min. In parallel, human alpha-thrombin was purified on a Micro Bio-Spin P-6 gel column (Biorad, Munich, Germany) which had been equilibrated with 500 µl PBS, pH 7.4. After adding the thrombin solution the column was centrifugated at 1000 x g for 2 min. Subsequently, 50 µl of purified thrombin (5 µM) were mixed with 50 µl of the solution of modified aptamer and incubated at room temperature to allow for the formation of covalent complexes. Afterwards, the coupled aptamer-thrombin complexes were purified from unbound compounds by centrifugation at 1000 x g for 4 min at 4°C on a Micro Bio-Spin P-30 gel column (Biorad, Munich, Germany).

### Example 2

### Reversal of anticoagulant activity of dabigatran by human α-thrombin

To confirm that thrombin acts as an antidote to direct acting thrombin inhibitors in-vitro reversal studies in dabigatran-containing human plasma were performed.

To human plasma obtained from healthy blood donors dabigatran was added to achieve a concentration of 250 ng/ml. A plasma concentration of 280 ng/ml dabigatran represents the upper limit of the peak level reached after 2-4 hours after dabigatran administration in humans. Samples of 50 µL of dabigatran containing plasma were mixed with concentrations of 11 nM, 22 nM, 44 nM, 88 nM, and 176 nM human α-thrombinand a blank without thrombin. After 1 minute of incubation, the concentration of the remaining anticoagulant active dabigatran was measured through determination of the anti-thrombin activity by a direct thrombin inhibitor assay as described above. Data were acquired in three independent experiments.

The Figure 1 shows the reversal of the anticoagulant activity of dabigatran using 11 nM, 22 nM, 44 nM, 88 nM, and 176 nM of human α-thrombin.As can be seen in the Figure 1, the addition of purified thrombin to dabigatran-containing plasma resulted in a concentration-dependent decrease in the dabigatran-induced anticoagulant effect. With increasing concentrations of thrombin the concentration of the remaining anticoagulant active dabigatran decreased linearly. To lower the plasma concentration of anticoagulant active dabigatran from 280 ng/ml representing the upper limit of the therapeutic plasma level, approximately 100 nM thrombin are required. This experiment shows that thrombin would function as an effective antidote against direct acting thrombin inhibitors. However, infusion of functional thrombin at this level bears a high risk to induce local and/or systemic activation of the hemostatic system leading to thromboembolic complications and/or uncontrolled bleeding due to consumption of clotting factors and overwhelming formation of activated protein C.

### Example 3

### Inhibition of coagulant activity of the aptamer-thrombin complex (aptathrombin)

To explore the safety of the aptamer-thrombin complex (aptathrombin), the release of fibrinopeptide A after addition of aptathrombin was monitored in citrate anti-coagulated plasma. In normal human plasma and whole blood, the addition of thrombin to plasma converts fibrinogen into fibrin as is characterized by the release of the fibrinopeptide A.

Plasma obtained from healthy blood donors was anticoagulated with 0.109 M buffered citrate. To samples of 50 µl of citrate anti-coagulated plasma were added 11 nM, 22 nM, 44 nM, 88 nM, and 176 nM of human α-thrombinand aptamer-thrombin complex (aptathrombin), respectively. The formation of fibrinopeptide A from fibrinogen in the samples was measured by by a commercially available ELISA (IMUCLONE™ FPA (Fibrinopeptide A) ELISA (Sekisui / American Diagnostica, Pfungstadt, Germany).

The Figure 2 shows the formation of fibrinopeptide A from fibrinogen in citrate anti-coagulated plasma samples upon addition of 11 nM, 22 nM and 44 nM of human α-thrombin and 11 nM, 22 nM and 44 nM, 88 nM, and 176 nM of aptamer-thrombin complex. As can be seen in the Figure 2, the addition of low nanomolar concentrations of 11 nM, 22 nM and 44 nM of thrombin induced a rapid increase of fibrinopeptide A levels to 123 mM, 256 mM, and 1345 mM. A release of fibrinopeptide A was not detectable when aptamer-protected thrombin was added even at high nanomolar concentrations.

This experiment shows that no prothrombotic response is induced in vitro by the aptamer-thrombin complex according to the invention. This finding confirms that the aptamer inhibits the pro-coagulant activity of thrombin in the aptamer-thrombin complex according to the invention.

### Example 4

### In vitro antagonisation of direct thrombin inhibitors using the aptamer-thrombin complex

The in-vitro antidote properties of aptamer-thrombin complex (aptathrombin) were studied against the direct thrombin inhibitors argatroban and dabigatran.

To human plasma obtained from healthy blood donors dabigatran or argatroban was added to achieve a concentration of 250 ng/ml or 500 ng/ml, respectively. Samples of 50 µL of dabigatran or argatroban containing plasma were mixed with concentrations of 44 nM or 88 nM of aptathrombin or blank. For quantification of the anticoagulant effect of the DTIs after addition of aptathrombin, the thrombin clotting time was measured 60s after addition of aptathrombin as described above.

The Figure 3 shows the dose-dependent reversal of the anticoagulant activity of argatroban and dabigatran in plasma by addition of 44 nM and 88 nM of aptathrombin, measured as a decrease of the thrombin clotting time. As can be seen in the Figure 3, 44 nM and 88 nM of aptathrombin significantly lowered the anticoagulant activity of argatroban and dabigatran. This shows that the aptamer-thrombin complex according to the invention is capable of neutralizing the activity of direct acting thrombin inhibitors and able to reverse the effects of anticoagulation in vitro in human plasma.

### Example 5

### In vivo antagonisation of dabigatran in a mouse model

The in-vivo antidote properties of aptathrombin were studied in a mouse model after oral administration of 1 mg/g body weight dabigatran. Anticoagulation was monitored and it was observed that feeding of mice with dabigatran resulted in detectable anti-FIIa activities reaching a peak level after 3-4 hours indicating a nearly identical pharmacokinetic profile as in humans.

Twelve weeks old male healthy C57BL/6-mice were fed with 1 mg/g body weight dabigatran. Three to four hours after dabigatran administration five mice were treated intravenously with an i.v. bolus of 480 nM aptathrombin in 200 µL of 0.9% NaCl, and three control mice received buffer. At 2, 7, 10 and 20 minutes following aptathrombin administration blood samples were taken and the residual blood levels of anticoagulant active dabigatran was measured using the direct thrombin inhibitor assay as described above.

The Figure 4 shows the level of anticoagulant activity of dabigatran determined for 20 min following aptathrombin injection. As can be seen the Figure 4, contrary to the controls, administration of aptathrombin immediately lowered the plasma level of anticoagulant active dabigatran demonstrating the rapid onset of the reversing effect. About 7 minutes after aptathrombin administration the dabigatran concentration increased again. It is assumed that this increase was due to a redistribution of dabigatran from the extravascular space. This demonstrates the efficacy and safety of the aptamer-thrombin complex according to the invention in a mouse model.

### Example 6

### Determination of the dose-effect relationship

Twelve weeks old male C57BL/6-mice were fed with 1 mg/g body weight dabigatran. Three to four hours after dabigatran administration mice were treated intravenously with an i.v. bolus of 80 nM, 240 nM, 480 nM or 1 µM of aptathrombin in a volume of 200 µL of 0.9% NaCl. Control mice received buffer without aptathrombin. Two minutes and 7 minutes following aptathrombin administration, blood samples were taken and the residual blood levels of anticoagulant active dabigatran was measured using the direct thrombin inhibitor assay as described above. For each determination two mice were taken.

The Figure 5 shows the level of anticoagulant active dabigatran after 2 minutes and 7 minutes following injection of aptathrombin of increasing dose. As can be seen the Figure 5, a linear relationship was determined between the decrease of anticoagulant activity of dabigatran and increasing concentration of aptathrombin. The effect was more pronounced 2 minutes compared to 7 minutes after administration. This demonstrates a linear dose-effect relationship of the aptamer-thrombin complex according to the invention in vivo.

### Example 7

### Determination of the effect of repeated administration of a bolus of aptathrombin

It was determined if a second bolus administration after an initial bolus achieves an intensification of the antidote effect. Twelve weeks old male C57BL/6-mice were fed with 1 mg/g body weight dabigatran. Three to four hours after dabigatran administration (n=4) mice were treated intravenously with a bolus of 480 nM aptathrombin in 200 µL of 0.9% NaCl. Ten minutes after the initial bolus, the mice received a second bolus at the same dosage of aptathrombin. Control mice (n=2) received at t=0 and t=10 min, respectively, buffer without aptathrombin. At t=0, and 2, 7, 10, 12, 17, and 20 minutes following the initial aptathrombin administration, blood samples were taken and the residual blood levels of anticoagulant active dabigatran was measured using the direct thrombin inhibitor assay as described above.

The Figure 6 shows the level of anticoagulant active dabigatran of dabigatran anticoagulated mice which received two administrations aptathrombin in an interval of 10 minutes at t=0 and t=10 min, and control mice which received only buffer for a time period of 20 minutes. As can be seen in the Figure 6, the second administration of aptathrombin results in a further lowering of the level of anticoagulant active dabigatran. The control mice showed no significant changes in anticoagulant active dabigatran level.

This demonstrates an additive reduction of the anticoagulant activity of dabigatran in blood plasma after repeated administration of the aptamer-thrombin complex according to the invention in vivo.

## Claims

1. A complex formed by thrombin and a thrombin-binding aptamer comprising a nucleotide sequence selected from the group comprising 5'-GGTTGGTGTGGTTGG-AAAAAAAAAAAAAAAAGTCCGTGGTAGGGCAGGTTGGGGTGACT-3' (SEQ ID NO: 1) wherein optionally at least one of the adenosine nucleotides at positions 16 to 31 is replaced by a thymidine nucleotide and 5'-GGTTGGTGTGGTTGGTTTTTNNNNNNN-3' (SEQ ID NO: 2) wherein N represents a thymidine nucleotide or a deletion or pharmaceutically acceptable salts thereof capable of neutralizing the activity of a direct acting thrombin inhibitor.

2. The aptamer thrombin complex according to claim 1, wherein the direct acting thrombin inhibitor is selected from the group comprising dabigatran, dabigatran etexilate and/or argatroban.

3. The complex formed by thrombin and a thrombin-binding aptamer according to claims 1 or 2, wherein the aptamer of SEQ ID NO: 1 or SEQ ID NO: 2 and thrombin additionally are covalently linked.

4. The complex formed by thrombin and a thrombin-binding aptamer according to claims 1 to 3, wherein the aptamer has a nucleotide sequence selected from the group comprising 5'-GGTTGGTGTGGTTGGAAAAAAATAAAAAAAAGTCCGTGGTAGGGCAGGTTG-GGGTGACT-3' (SEQ ID NO: 3) and 5'-GGTTGGTGTGGTTGGTTTTTTTTTT-3' (SEQ ID NO: 4).

5. A complex formed by thrombin and a thrombin-binding aptamer comprising a nucleotide sequence selected from the group comprising 5'-GGTTGGTGTGGTTGG-AAAAAAAAAAAAAAAAGTCCGTGGTAGGGCAGGTTGGGGTGACT-3' (SEQ ID NO: 1) wherein optionally at least one of the adenosine nucleotides at positions 16 to 31 is replaced by a thymidine nucleotide and 5'- GGTTGGTGTGGTTGGTTTTTNNNNNNN-3' (SEQ ID NO: 2) wherein N represents a thymidine nucleotide or a deletion, or a pharmaceutically acceptable salt thereof, for use as a medicament.

6. A complex formed by thrombin and a thrombin-binding aptamer comprising a nucleotide sequence selected from the group comprising 5'-GGTTGGTGTGGTTGG-AAAAAAAAAAAAAAAAGTCCGTGGTAGGGCAGGTTGGGGTGACT-3' (SEQ ID NO: 1) wherein optionally at least one of the adenosine nucleotides at positions 16 to 31 is replaced by a thymidine nucleotide, 5'-GGTTGGTGTGGTTGGTTTTTNNNNNNN-3' (SEQ ID NO: 2) wherein N represents a thymidine nucleotide or a deletion and 5'-GGTTGGTGTGGTTGG-3' (SEQ ID NO: 5) or pharmaceutically acceptable salts thereof, for use as an antidote to blood anticoagulants selected from direct acting thrombin inhibitors.

7. The complex formed by thrombin and a thrombin-binding aptamer for use according to claim 6, wherein the complex is for use in the therapeutic and/or prophylactic treatment of a bleeding or overdosing event in anticoagulant therapy.

8. The complex formed by thrombin and a thrombin-binding aptamer for use according to claim 6 or 7, wherein the direct acting thrombin inhibitor is selected from the group comprising dabigatran, dabigatran etexilate and/or argatroban.

9. The complex formed by thrombin and a thrombin-binding aptamer for use according to claims 6 to 8, wherein the aptamer has a nucleotide sequence selected from the group comprising 5'-GGTTGGTGTGGTTGGAAAAAAATAAAAAAAAGTCCGTGGTA-GGGCAGGTTGGGGTGACT-3' (SEQ ID NO: 3) and 5'-GGTTGGTGTGGTTGGTTTTTTTTTT-3' (SEQ ID NO: 4).

10. A pharmaceutical composition comprising as an active ingredient a complex formed by thrombin and a thrombin-binding aptamer comprising a nucleotide sequence selected from the group comprising 5'-GGTTGGTGTGGTTGGAAAAAAAAAAAAAAAA-GTCCGTGGTAGGGCAGGTTGGGGTGACT-3' (SEQ ID NO: 1) wherein optionally at least one of the adenosine nucleotides at positions 16 to 31 is replaced by a thymidine nucleotide and 5'-GGTTGGTGTGGTTGGTTTTTNNNNNNN-3' (SEQ ID NO: 2) wherein N represents a thymidine nucleotide or a deletion or a pharmaceutically acceptable salt thereof.

11. A pharmaceutical composition comprising as an active ingredient a complex formed by thrombin and a thrombin-binding aptamer comprising a nucleotide sequence selected from the group comprising 5'-GGTTGGTGTGGTTGGAAAAAAAAAAAAAAAA-GTCCGTGGTAGGGCAGGTTGGGGTGACT-3' (SEQ ID NO: 1) wherein optionally at least one of the adenosine nucleotides at positions 16 to 31 is replaced by a thymidine nucleotide, 5'-GGTTGGTGTGGTTGGTTTTTNNNNNNN-3' (SEQ ID NO: 2) wherein N represents a thymidine nucleotide or a deletion and 5'-GGTTGGTGTGGTTGG-3' (SEQ ID NO: 5) or pharmaceutically acceptable salts thereof, for use as an antidote to blood anticoagulants selected from direct acting thrombin inhibitors.

12. The pharmaceutical composition according to claim 11, wherein the composition is for use in the therapeutic and/or prophylactic treatment of a bleeding or overdosing event in anticoagulant therapy.

13. Use of a complex formed by thrombin and a thrombin-binding aptamer comprising a nucleotide sequence selected from the group comprising 5'-GGTTGGTGTGGTTGG-AAAAAAAAAAAAAAAAGTCCGTGGTAGGGCAGGTTGGGGTGACT-3' (SEQ ID NO: 1) wherein optionally at least one of the adenosine nucleotides at positions 16 to 31 is replaced by a thymidine nucleotide and 5'-GGTTGGTGTGGTTGGTTTTTNNNNNNN-3' (SEQ ID NO: 2) wherein N represents a thymidine nucleotide or a deletion or a pharmaceutically acceptable salt thereof, for the manufacture of a medicament.

14. Use of a complex formed by thrombin and a thrombin-binding aptamer comprising a nucleotide sequence selected from the group comprising 5'-GGTTGGTGTGGTTGG-AAAAAAAAAAAAAAAAGTCCGTGGTAGGGCAGGTTGGGGTGACT-3' (SEQ ID NO: 1) wherein optionally at least one of the adenosine nucleotides at positions 16 to 31 is replaced by a thymidine nucleotide, 5'-GGTTGGTGTGGTTGGTTTTTNNNNNNN-3' (SEQ ID NO: 2) wherein N represents a thymidine nucleotide or a deletion and 5'-GGTTGGTGTGGTTGG-3' (SEQ ID NO: 5) or pharmaceutically acceptable salts thereof, for the manufacture of a medicament for use as an antidote to blood anticoagulants selected from direct acting thrombin inhibitors.

15. A method for preventing or treating a bleeding or overdosing event in anticoagulant therapy, the method comprising the step of administering to a subject a therapeutically effective amount of a complex formed by thrombin and a thrombin-binding aptamer comprising a nucleotide sequence selected from the group comprising 5'-GGTTGGTGTGGTTGGAAAAA AAAGTCCGTGGTAGGGCA-GGTTGGGGTGACT-3' (SEQ ID NO: 1) wherein optionally at least one of the adenosine nucleotides at positions 16 to 31 is replaced by a thymidine nucleotide, 5'-GGTTGGTGTGGTTGGTTTTTNNNNNNN-3' (SEQ ID NO: 2) wherein N represents a thymidine nucleotide or a deletion and 5'-GGTTGGTGTGGTTGG-3' (SEQ ID NO: 5) or pharmaceutically acceptable salts thereof.
